# EUROPÄISCHE PATENTANMELDUNG

(11) **EP 2 497 465 A2**
(43) Veröffentlichungstag der Anmeldung: **12.09.2012**
(21) Anmeldenummer: 12170347.4
(22) Anmeldetag: 27.05.2008
(51) Int. Cl.: A61K 9/24, A61K 9/44, A61K 9/00

(54) **Gastroretentives System mit Alginat-Körper**

(30) Priorität: 04.06.2007 DE 102007026037
(62) Teilanmeldung aus: 08758774.7
(71) Anmelder: LTS LOHMANN Therapie-Systeme AG, 56626 Andernach (DE)
(72) Erfinder: Asmussen, Bodo, 56170 Bensdorf (DE); Schiller, Christiane, 18435 Stralsund (DE); Weitschies, Werner, 17498 Neuenkirchen (DE)
(74) Vertreter: Michalski Hüttermann & Partner

(57) **Zusammenfassung**

Die Erfindung betrifft gastroretentive Systeme, umfassend mindestens eine Abgabeeinrichtung für mindestens einen pharmazeutischen Wirkstoff, und mindestens einen Quellkörper, der mit der Abgabeeinrichtung verbunden ist.

## Beschreibung

Die Erfindung betrifft ein oral applizierbares gastroretentives System zur gesteuerten, kontinuierlichen Freisetzung mindestens eines pharmazeutischen Wirkstoffs (= Arzneistoff) im Magen, umfassend mindestens eine Abgabeeinrichtung und mindestens einen fest mit der Abgabeeinrichtung verbundenen Quellkörper, wobei die Abgabeeinrichtung(en) und der/die Quellkörper unabhängig voneinander funktionieren können.

Ein Ziel bei der Entwicklung von Arzneimitteln ist, Arzneiformen bereitzustellen, mit denen ein über mehrere Stunden konstant hoher Wirkstoffspiegel im Körper des Patienten aufrecht erhalten werden kann. Mit schnell zerfallenden Tabletten ist dies jedoch nicht möglich, da sie den in ihnen enthaltenen Wirkstoff auf einmal freisetzen. Daher wurden tablettenförmige Arzneimittel entwickelt, die den in ihnen enthaltenen Arzneistoff gesteuert und über einen längeren Zeitraum hinweg kontinuierlich freisetzen können.

So wird in US 5,296,233 ein kapselartiges, zweifach unterschichtetes Medikament beschrieben, das einen festen, wirkstoffhaltigen Kapselkern und eine zweifache Unterbeschichtungszusammensetzung umfasst. Die zweifache Unterbeschichtungszusammensetzung umfasst eine erste Unterbeschichtung, die ein wasserlösliches, filmbildendes Polymer aufweist, beispielsweise Povidon, und auf den Kapselkern aufgetragen ist, sowie eine zweite Unterbeschichtung, die ihrerseits eine Mischung aus mindestens einem wasserlöslichen, filmbildenden Polymer und einem hydrophoben Weichmacher, beispielsweise Rizinusöl, umfasst. Ferner weist das Medikament eine glatte, gleichmäßige und substantiell blasenfreie äußere Beschichtung auf, damit das Medikament trotz seines vergleichsweise großen Volumens problemlos schluckbar ist.

US 4,983,401 beschreibt ein pharmazeutisches Präparat zur verzögerten Wirkstofffreisetzung unter Verwendung einer pH-kontrollierbaren Diffusionsmembran, bestehend aus einem pH-sensitiven, filmbildenden Polymer. Dieses kann Phthalsäuregruppen enthalten, die mit einer ihrer Carboxylgruppen über eine Esterbindung an das Ausgangspolymer gebunden sind, während die zweite Carboxylgruppe als freie Säure frei bleibt, so dass das modifizierte Filmbildnerpolymer bei niedrigem pH-Wert hydrophob und bei höherem pH-Wert hydrophil ist.

In EP 0 259 219 A2 wird eine umhüllte Tablette mit einer zentralen Öffnung beschrieben, durch welche der Wirkstoff aus einem erodierbaren Tablettenkern nach außen freigesetzt wird. Die Dicke des Tablettenkerns nimmt von der zentralen Öffnung zur Peripherie hin zu, wodurch sich die im Freisetzungsverlauf vergrößernde Distanz zwischen Erosionsfront und Öffnung durch eine Flächenvergrößerung kompensiert wird.

Aus der EP 0 542 364 A1 ist eine Vorrichtung zur kontrollierten Freisetzung wenigstens eines Wirkstoffes in ein flüssiges Medium bekannt, die in Form einer Tablette vorliegt. Diese Vorrichtung umfasst eine sowohl für den Wirkstoff als auch für das Medium undurchlässige Umhüllung, die wenigstens eine Öffnung aufweist und einen Hohlraum begrenzt, der von einem wirkstofflialtigen Kern ausgefüllt wird, wobei der Kern an die Öffnung heranreicht. Die geometrische Form und die Formulierung der Tablette sind derart, dass der Wirkstoff über einen signifikanten Zeitraum hinweg mit einer konstanten Rate abgegeben werden kann.

EP 0 779 807 A1 offenbart eine Manteltablette zur kontrollierten Freisetzung von Wirkstoffen, die einen erodierbaren, mindestens einen Wirkstoff enthaltenden Innenkern und eine weitgehend erosionsresistente Mantelschicht aufweist, welche eine Umhüllung bildet und wenigstens eine Öffnung aufweist. Der Innenkern ist zu seinem Endbereich hin spitz oder schmal zulaufend geformt und so in der Tablette plaziert, dass sein spitzer bzw. schmaler Endbereich an den Außenrand der Manteltablette heranreicht und an dieser Stelle die den Kern umgebende Masse der Mantelschicht unterbricht, so dass die am Tablettenaußenrand befindliche Öffnung ausbildet wird.

EP 0 797 429 A1 beschreibt eine osmotische Vorrichtung zur kontinuierlichen Freisetzung von Wirkstoffen in Flüssigkeiten des Gastrointestinaltraktes, bei der der Wirkstoff durch eine in der äußeren Membran der Vorrichtung angeordnete Austrittsöffnung freigesetzt wird. Die Austrittsöffnung ist so an einer relativ zurückliegenden Stelle der äußeren Membran angeordnet, dass sie nicht mit der Schleimhautoberfläche in Kontakt kommen kann.

Auch bei diesen oral zu verabreichenden Darreichungsformen besteht ein Nachteil darin, dass der Plasmaspiegel des Arzneistoffs bei den Patienten unterschiedlich hoch sein kann und schlecht vorhersagbar ist. Dies kann daran liegen, dass sich die Absorbierbarkeit des Arzneistoffs in unterschiedlichen Bereichen des Magen-Darm-Traktes stark ändert und die verabreichte Darreichungsform bei unterschiedlichen Patienten mit unterschiedlicher Geschwindigkeit durch den Magen-Darm-Trakt transportiert wird. Man spricht im Zusammenhang mit der sich ändernden Absorbier-barkeit eines Arzneistoffs entlang des Magen-Darm-Traktes auch von "Resorptions-fenstern". Beispielsweise werden einige Arzneistoffe bevorzugt in Darmabschnitten resorbiert, die sich nah beim Magenausgang befinden. Direkt an den Magenausgang schießt sich der etwa 20-30 cm lange Zwölffingerdarm (Duodenum) an, der für eine Vielzahl von Arzneistoffen die höchste Resorptionskapazität bietet. In weiter vom Magenausgang entfernten, tiefer liegenden Darmabschnitten werden diese Arzneistoffe in der Regel nur noch in geringer Menge aufgenommen. Da es derzeit nicht möglich ist, verlässliche Aussagen über die Transitgeschwindigkeit einer Arzneiform bei Patienten zu machen, kann nicht vorhergesagt werden, wann sich eine Arzneiform nach ihrer oralen Verabreichung in einem für die Resorption des in ihr enthaltenen Arzneistoffs günstigen Bereich des Magen-Darm-Traktes befindet.

Um einen langanhaltenden, konstanten Plasmaspiegel eines Arzneistoffs erreichen zu können, der im Magen-Darm-Trakt resorbiert wird, vorzugsweise sein Resorptionsfenster im Duodenum bzw. oberen Dünndarmabschnitt hat, stellen feste Arzneiformen mit einer verlängerten Verweilzeit im Magen einen vielversprechenden Ansatz zur Lösung der geschilderten Probleme dar. Derartige Arzneiformen werden auch als "Gastroretentive Systeme" bezeichnet.

Gastroretentive Systeme, also Arzneiformen, die eine längere Verweildauer im Magen haben als Kapseln oder Tabletten, sind an sich bekannt. Ohne einen Arzneistoff zu enthalten dienen sie der zumindest teilweisen Füllung des Magens, um ein Sättigungsgefiihl zu erzeugen und so eine Gewichtsreduzierung zu erreichen. Arzneistoffhaltige gastroretentive Systeme ermöglichen eine retardierende Abgabe von Arzneistoffen im Magen.

Gastroretentive Systeme sollen hinsichtlich ihrer Einnahmevorgaben denen herkömmlicher fester oraler Arzneiformen weitgehend entsprechen, damit die bekannten Einnahmegewohnheiten beibehalten werden können. Sie sollen eine annehmbare Größe haben und gut zu schlucken sein. Darüber hinaus sollen die gastroretentiven Systeme ausreichend lange im Magen verweilen und dort den in ihnen enthaltenen Arzneistoff kontrolliert abgeben. Nach Erfüllung dieser Aufgabe soll das gastroretentive System entweder im Magen-Darm-Trakt abgebaut werden oder ihn auf natürlichem Wege sicher verlassen.

Hierfiir wurden gastroretentive Vorrichtungen entwickelt, die eine geringere Dichte als der Mageninhalt aufweisen. Diese Vorrichtungen sollen aufgrund ihres Auftriebs auf der Magenflüssigkeit bzw. dem Mageninhalt flotieren. Beispielsweise wurden in der WO 02/85332 A1 Vorrichtungen beschrieben, die einen hohen Anteil an lipophilen Substanzen mit geringer Dichte enthalten.

EP 0 326 816 A offenbart eine schwimmfähige Wirkstoff-Dosierform, die eine lange Aufenthaltsdauer im Gastrointestinaltrakt gewährleistet und bei der Hohlräume von zumindest einem Strukturelement eingeschlossen werden. Insbesondere kann das Strukturelement eine geschäumte oder mikroporöse Polymermatrix sein, beispielsweise von Polyolefin, Polyamid, Polyester, Polystyrol, Polyacrylat, Polytetrafluorethylen (PTFE), Polyvinylchlorid (PVC), Polyvinylidenchlorid oder Polysiloxan, gegebenenfalls in Form eines faltbaren oder rollbaren Films, Tablettenkerns oder in Schichtform. Alternativ können die Strukturelemente hohle Partikel aus z.B. Glas oder Keramik sein, die in eine Wirkstoff enthaltende Matrix-Komposition eingelagert sind, insbesondere zur Verwendung in Kapseln. Die Gebilde können mit einer die Freisetzung von Wirkstoff kontrollierenden Membran versehen sein.

Ein anderer Lösungsweg, eine längere Verweildauer im Magen zu erreichen, wurde durch die Entwicklung von gastroretentiven Systemen beschritten, die aufgrund ihrer Größe oder Gestalt den Magenpförtner (Pylorus) nicht passieren und somit den Magen nicht verlassen können. Diese Vorrichtungen liegen in der Regel komprimiert vor und entfalten sich erst nach Kontakt mit der Magenflüssigkeit zu ihrer vorgesehenen Größe.

Beispielsweise offenbart die WO 02/00213 A1 eine gastroretentive Arzneiform mit einer sich schnell ausdehnenden Zubereitung, die aus einem sich sehr schnell zersetzenden Stoff, Gerbsäure und mindestens einem Hydrogel besteht.

Auch mit der WO 2005/079384 A2 wird eine expansionsfähige gastroretentive Vorrichtung offenbart, die ein getrocknetes Polysaccharid-Gel umfasst, in dem ein Arzneistoff enthalten sein kann.

In der WO 01/97783 A1 wird auf den Einfluss der Größe und der Quelleigenschaften eines gastroretentiven Systems auf dessen Verweildauer im Magen eingegangen.

Bekannt sind darüber hinaus gastroretentive Systeme, die aufgrund einer Volumenvergrößerung durch Gaserzeugung über einen längeren Zeitraum im Magen zurückgehalten werden und auch auf dem Mageninhalt flotieren.

Beispielsweise bei einer Darreichungsform gemäß US 4,996,058, bei der sich der Wirkstoff zusammen mit einer Verbindung, die bei Kontakt mit Magenflüssigkeit

Kohlendioxid oder Stickstoff erzeugt, in einem geschlossenen Beutels aus einer hydrophilen Membran befindet. Der Wirkstoff wird bei diesem gastroretentiven System durch den in den Beutel eindringenden Magensaft aufgelöst und kontrolliert durch die Membran des Beutels abgegeben, die die Freisetzung des Wirkstoffs kontrolliert.

Solche Systeme sind z.B. in US 4,207,890 und DE 44 19 818 A1 beschrieben. Ihre Wirkungsweise beruht im Wesentlichen darauf, dass ein Beutel mit Wirkstoff gefüllt wird, der sich innerhalb eines definierten Zeitraums auflöst und in kontrollierter Weise in die Umgebung freigesetzt wird. Die Volumenvergrößerung wird dadurch erreicht, dass sich innerhalb des Beutels auch geeignete, gaserzeugende Substanzen (wie z.B. Natriumhydrogencarbonat) oder gaserzeugende Substanzgemische befinden, die bei Eindringen des salzsäurehaltigen Magensafts in den Beutel durch chemische Reaktion ein Gas freisetzen, z.B. CO₂. Dabei wird der Beutel, der aus einer Membran besteht, quasi aufgeblasen und erreicht eine Größe, die die Passage des Systems in den Zwölffingerdarm verhindert.

Tritt der Magensaft in den Beutel, so wird gleichzeitig Wirkstoff freigesetzt. Dabei kann die Freisetzungscharakteristik des Wirkstoffs durch seine Form, z.B. als mikroverkapselte Partikel vorliegend, oder durch die Eigenschaften der Membran kontrolliert werden. Sobald der Wirkstoff durch die Membranwand diffundiert und somit in den Mageninhalt gelangt ist, kann er über die Magenschleimhaut oder die Darmwand resorbiert werden. Da das System über einen längeren Zeitraum im Magen verweilt, erfolgt die kontrollierte Wirkstofffreisetzung im Magen über diesen längeren Zeitraum, vorzugsweise bis zu 24 Stunden.

Gastroretentive Vorrichtungen, die Kohlendioxid erzeugende Bestandteile enthalten, werden auch in WO 03/011255 A1 und US 2006/003003 A1 beschrieben.

Das Funktionieren der flotierenden Systeme ist jedoch von der im Magen vorhandenen Flüssigkeitsmenge abhängig. Damit derartige Arzneiformen überhaupt schwimmen können, muss eine Mindestmenge an Mageninhalt bzw. Magensaft im Magen vorhanden sein. Bei nüchternen Probanden befanden sich jedoch nur durchschnittlich 20 bis 50 ml Flüssigkeit im Magen. Ein stabiles Flotieren und damit ein durch Auftrieb bedingtes Vermeiden, den Magen zu verlassen, dürften bei nüchternen Patienten somit kaum realisierbar sein. Dies dürfte auch ein Grund für die Unzuverlässigkeit derartiger Systeme sein.

Ein weiterer Lösungsweg wurde dadurch beschritten, dass die Aufgabe der Freisetzung des Arzneistoffs von der Aufgabe der Ortsbeständigkeit des Systems im Magen getrennt wurde. Solche dualen Systeme, bestehend aus einer arzneistoffhaltigen Matrix und einer Quellschicht werden beispielsweise in US 2005/0019409 A1 und US 2006/0013876 A1 beschrieben. Nachteilig bei derartigen Systemen ist jedoch, dass die Freisetzung des Arzneistoffs aus der Matrix diffusionsgesteuert erfolgt und gegebenenfalls von den Umgebungsbedingungen wie Mageninhalt, pH-Wert, Ionenstärke und Druck abhängig ist.

Die der vorliegenden Erfindung zugrundeliegende Aufgabe bestand darin, ein gastroretentives System für eine kontrollierte Arzneistoffverabreichung über einen längeren Zeitraum zu entwickeln, das die Nachteile der aus dem Stand der Technik bekannten gastroretentiven Systeme nicht aufweist.

Die Aufgabe wird durch ein gastroretentives System gelöst, das zwei Elemente umfasst, die unabhängig voneinander funktionieren, jedoch fest miteinander verbunden sind. Das erste Element (Element A) ist mindestens ein Quellkörper, der die Verweildauer des Systems im Magen verlängert und vorzugsweise auf einem Natriumalginat basiert. Das zweite Element (Element B) ist mindestens eine Abgabeeinrichtung für den Arzneistoff, die eine gesteuerte Freisetzung des Arzneistoffs ermöglicht, beispielsweise eine osmotisch gesteuerte oder eine erosionsgesteuerte Freisetzung.

Gegenstand der Erfindung ist somit ein gastroretentives System, umfassend mindestens einen Quellkörper und mindestens eine Vorrichtung für die Abgabe mindestens eines Arzneistoffs, wobei der/die Quellkörper und die Abgabeeinrichtung(en) fest miteinander verbunden sind, aber unabhängig voneinander funktionieren können.

Das erfindungsgemäße gastroretentive System kann somit Ausführungsformen mit nur einem Quellkörper oder aber auch mit mehreren Quellkörpern umfassen. Ebenso kann es Ausführungsformen mit nur einer Abgabeeinrichtung oder mit mehreren Abgabeeinrichtungen umfassen, mit denen derselbe Arzneistoff oder verschiedene Arzneistoffe verabreicht werden können. Selbstverständlich gelten die nachfolgenden Ausführungen bezüglich Quellkörper und Abgabeeinrichtung für alle Ausführungsformen des erfindungsgemäßen gastroretentiven Systems, auch wenn nachfolgend Ausdrucksweisen im Singular verwendet werden.
Figur 1 ist eine Graphik, die die relative Masseänderung von Quellkörpern auf Basis von Natriumalginat in einem wässrigen Medium mit einem pH-Wert von 3 veranschau-licht.
Figur 2 ist eine Graphik, die die relative Masseänderung von Quellkörpern auf Basis von Natriumalginat in einem wässrigen Medium mit einem pH-Wert von 4,5 veran-schaulicht.
Figur 3 ist eine Graphik, die die relative Masseänderung von Quellkörpern auf Basis von Natriumalginat in einem wässrigen Medium veranschaulicht, dessen pH-Wert schrittweise erhöht wurde.

Der Quellkörper gewährleistet eine längere Verweildauer des Systems im Magen, indem er nach Applikation im Magen quillt. Vorzugsweise basiert der Quellkörper auf Natriumalginat, das sich durch seine guten Quelleigenschaften auszeichnet. Nach Einbringen in den Magen kann sich der Quellkörper zu seiner vollständigen Größe entfalten. Im Milieu des menschlichen Darms löst sich der Quellkörper rasch auf, so dass nach Entleerung aus dem Magen eine Kumulation im Darm und damit ein eventuell drohender Darmverschluss vermieden werden kann.

Bei einer bevorzugten Ausführungsform wird dem Natriumalginat ein pharmazeutisch annehmbares Calciumsalz zugesetzt oder eine Mischung aus Natriumalginat und Calciumalginat verwendet. Natriumalginat zeigt sich nur bei pH-Werten von 1 bis 2 ausreichend stabil, löst sich im Milieu des menschlichen Darms jedoch relativ schnell auf. Der Zusatz eines Calciumsalzes stabilisiert die Quelleigenschaften des Quellkörpers oder Natriumalginat-Körpers bei höheren pH-Werten als den zuvor genannten. Überraschenderweise bleiben die aus Sicherheitsgründen erforderlichen Zerfallseigen-schaften des Quellkörpers im "neutralen" Darmmilieu erhalten, auch wenn ihm Calciumionen oder Calciumalginat zugesetzt wurden.

Der Zusatz von Calciumionen kann prinzipiell durch den Zusatz eines beliebigen, pharmazeutisch annehmbaren Calciumsalzes oder einer Mischung aus zwei oder mehreren dieser Calciumsalze erfolgen. Pharmazeutisch annehmbare Calciumsalze sind beispielsweise Calciumacetat, Calciumaspartat, Calciumcarbonat, Calciumchlorid, Calciumcitrat, Calciumcyclamat, Calciumfolinat, Calciumgluconat, Calciumglutamat, Calciumlactat, Calciumlactatgluconat, Calciumphosphat und Calciumsulfat.

Der Anteil an Calciumionen im Quellkörper, bezogen auf die Masse des Quellkörpers, kann zwischen 0,1 und 10 Gew.-% liegen. Vorzugsweise beträgt er zwischen 0,3 und 8 Gew.-%, besonders bevorzugt zwischen 0,5 und 5 Gew.-%. Ganz besonders bevorzugt weist der Quellkörper einen Anteil an Calciumionen von 0,6 bis 2 Gew.-% auf.

Alternativ oder zusätzlich zur Zugabe von Calciumionen können auch Zink- und/oder Aluminium-Ionen in Form pharmazeutisch annehmbarer Salze verwendet werden.

Pharmazeutisch annehmbare Zinksalze sind beispielsweise Zinkacetat, Zinkaspartat, Zinkbishydrogenaspartat, Zinkchlorid und Zinkgluconat. Aus pharmazeutischer Sicht einsetzbare Aluminiumsalze sind zum Beispiel Aluminiumhydroxid, Algedrat (47 - 60% Aluminiumoxid (Al₂O₃)) und Aluminiumphosphat.

Der Anteil an Zinksalz(en) oder Aluminiumsalz(en) im Quellkörper liegt vorzugsweise zwischen 0,1 und 30 Gew.-%, besonders bevorzugt zwischen 1 und 25 Gew.-% und ganz besonders bevorzugt zwischen 5 und 15 Gew.-%, bezogen auf die Masse des Quellkörpers.

Es ist auch möglich, Mischungen des Natriumalginats mit weiteren Polymeren, die ebenfalls Quelleigenschaften haben, zu verwenden, sofern die pH-abhängigen Zerfallseigenschaften des Quellkörpers erhalten bleiben. Beispielsweise sind Mischungen von Natriumalginat mit Croscarmellose-Natrium, Polycarbophil (Polyacrylat, das mit Divinylglykol vernetzt ist), Polyethylenoxid und/oder Cellulosederivaten zu nennen, wobei der Zusatz von Natrium-Croscarmellose besonders bevorzugt wird. Unter den Cellulosederivaten werden nicht wasserlösliche Cellulosederivate bevorzugt, insbesondere Ethylcellulose und Hydroxypropylmethylcellulose.

Der Anteil an weiteren Polymeren beträgt vorzugsweise 1 bis 30 Gew.-%, besonders bevorzugt 3 bis 20 Gew.-%, und ganz besonders bevorzugt 5 bis 15 Gew.-%, bezogen auf die Masse des Quellkörpers.

Für die Herstellung des Quellkörpers können zusätzlich weitere geeignete Hilfsstoffe eingesetzt werden, wie beispielsweise Fließregulierer, Schmier- oder Gleitmittel, Füllstoffe, Bindemittel und/oder Trennmittel. Als Füllstoffe können Stärkederivate, Zucker wie Sucrose oder Glucose, Zuckeraustauschstoffe wie Xylitol oder Sorbitol verwendet werden. Besonders bevorzugt werden Lactose oder mikrokristalline Cellulose verwendet. Als Bindemittel können Polyvinylpyrrolidon, Gelatine, Methylcellulose, Ethylcellulose, Gummi arabicum, Traganth, Polyethylenglykol, Stärkederivate verwendet werden. Einsetzbare Gleitmittel sind Magnesiumstearat, Calciumstearat, Calciumbehenat, Glycerinmonostearat, Stearinsäure und ihre Salze, Wachse, hochdisperses Siliciumdioxid und hydrierte Pflanzenfette.

Ferner können Stoffe zum Einsatz kommen, die den pH-Wert lokal in der Arzneiform beeinflussen können, z. B. Zitronensäure, Polycarbophil oder Algedrat.

Die Abgabeeinrichtung des erfindungsgemäßen gastroretentiven Systems ermöglicht eine Freisetzung des in ihr enthaltenen Arzneistoffs mit konstanter Rate über einen längeren Zeitraum hinweg, vorzugsweise während des gesamten Applikationszeitraums. Die Abgabe des Arzneistoffs erfolgt unabhängig von Umgebungsbedingungen, die eine diffusionsgesteuerte Arzneistoffabgabe beeinträchtigen würden, beispielsweise osmotisch gesteuert oder erosionsgesteuert.

Der Quellkörper und die Abgabeeinrichtung sind fest miteinander verbunden. Dazu können der Quellkörper und die Abgabeeinrichtung miteinander verpresst oder miteinander verklebt sein, so dass das erfindungsgemäße gastroretentive System beispielsweise in Form einer zwei- oder mehrschichtigen Tablette oder in Form einer Manteltablette vorliegen kann.

Als Klebstoff, um den Quellkörper und die Abgabeeinrichtung fest miteinander zu verbinden, werden physiologisch verträgliche, pharmazeutisch annehmbare Klebstoffe verwendet. Als Kleber können Polymere mit adhäsiven Eigenschaften eingesetzt werden. Hierzu zählen beispielsweise Acrylate, Methylmethacrylat-Polymere, Dextrin-basierte Kleber, Acrylat-Vinylacetat-basierte Klebstoffe, Carboxyvinylpolymere, Celluloseacetate und Ethylcellulosen.

Es besteht jedoch auch die Möglichkeit, den Quellkörper und die Abgabeeinrichtung innerhalb einer gemeinsamen, für Magenflüssigkeit wie für den Arzneistoff durchlässigen Hülle anzuordnen, beispielsweise innerhalb eines Netzes.

Bei einer Ausführungsform des erfindungsgemäßen gastroretentiven Systems ist die Abgabeeinrichtung ein osmotisches System, das in Form eines Einkammer-Systems vorliegen oder als Mehrkammer-System ausgebildet sein kann. Es kann auch in Form einer Ausführungsform vorliegen, bei dem räumlich getrennte Bereiche für den Arzneistoff und für die osmotisch wirksame Substanz nicht durch eine Membran voneinander getrennt sind.

Bei der Ausgestaltung der Abgabeeinrichtung als Einkammer-System ist ein arzneistoffhaltiger Kern von einer semipermeablen Polymermembran umhüllt, die eine kleine Öffnung aufweist. Der arzneistoffhaltige Kern dieser Abgabeeinrichtung wird in der Regel aus einer Mischung aus Arzneistoff und Hilfsstoffen als Tablette gepresst. Die Polymermembran kann durch ein Sprühverfahren auf den Kern aufgebracht werden, bei dem das Polymer mit weiteren Hilfsstoffen als Lösung oder Dispersion auf den Kern aufgetragen und anschließend getrocknet wird. Die Austrittsöffnung in der Polymermembran kann mit einem Laserstrahl erzeugt werden.

Nach der peroralen Applikation einer derartigen Abgabeeinrichtung als Bestandteil des erfindungsgemäßen gastroretentiven Systems diffundiert das in der Magenflüssigkeit enthaltene Wasser durch die Polymermembran in den arzneistoffhaltigen Kern. Der Arzneistoffund/oder ein dafiir vorgesehener, osmotisch aktiver Hilfsstoff beginnen sich in der eingedrungenen Flüssigkeit zu lösen. Hierdurch entsteht im Innenraum der Abgabeeinrichtung ein gegenüber dem Außenmedium erhöhter osmotischer Druck, der dazu führt, dass arzneistoffhaltige Lösung durch die Austrittsöffnung nach außen gedrückt wird. Weil sich das Nachdiffundieren von Flüssigkeit durch die Polymermembran in den Kern kontinuierlich fortsetzt, erfolgt der Druckausgleich durch Austritt von Arzneistofflösung mit großer Gleichmäßigkeit, solange Arzneistoff im Kern vorhanden ist. Die Geschwindigkeit der Arzneistoffabgabe lässt sich über die Zusammensetzung, Beschaffenheit und Ausdehnung der Membran sowie über die Löslichkeit der Bestandteile einstellen.

Soll ein Arzneistoff, der in dem Wasser der Magenflüssigkeit, das die Membran durchdringt, nur begrenzt löslich ist und daher keine ausreichende osmotische Wirkung ausübt, freigesetzt werden, können dem Arzneistoff eine oder mehrere osmotisch wirksame Substanzen zugesetzt werden. Als osmotisch wirksame Zusätze werden vorzugsweise Salze wie Natriumchlorid, Natriumcarbonat, Natriumsulfat, Natriumsulfit, Kaliumsulfat, Kaliumchlorid, saures Kaliumphosphat (KH₂PO₄), Calciumcarbonat, Calciumsulfat, Calciumlactat, Magnesiumsulfat, Magnesiumchlorid, Lithiumchlorid, Lithiumsulfat, D-Mannit, Harnstoff, Inosit, Weinsäure, Rohrzucker, Raffinose, Glucose oder α-D-Laktosemonohydrat verwendet. Sofern der Arzneistoff selbst hinreichend osmotisch wirksam ist, kann auf den Zusatz osmotisch wirksamer Substanzen verzichtet werden.

Die Polymermembran der Abgabeeinrichtung zur osmotisch gesteuerten Arzneistoff-freisetzung ist semipermeabel. Das bedeutet, dass sie für Wasser durchlässig, aber für gelöste Substanzen im Wesentlichen undurchlässig ist.

Zu den Materialien, die zur Herstellung semipermeabler Membranen für Abgabeeinrichtungen verwendet werden können, mit denen der Wirkstoff osmotisch gesteuert freigesetzt werden kann, gehören z. B. Celluloseacetat, Cellulosetriacetat, Agaracetat, Amylosetriacetat, β-Glucanacetat, β-Glucantriacetat, Acetaldehyddimethylacetat, Celluloseacetatmethylcarbamat, Celluloseacetatsuccinat, Celluloseacetatdimethaminoacetat, Celluloseacetatethylcarbonat, Celluloseacetatchloracetat, Celluloseacetatethyloxalat, Celluloseacetatmethylsulfonat, Celluloseacetatbutylsulfonat, Celluloseether, Celluloseacetatpropionat, Poly(vinylmethyl)ether-Copolymeren, Celluloseacetatdiethylaminoacetat, Celluloseacetacetat, Celluloseacetatlaurat, Methylcellulose, Celluloseacetatp-toluolsulfonat, Triacetat des Gummi arabicum, Celluloseacetat mit acetylierter Hydroxyethylcellulose, hydroxyliertes Ethlyenvinylacetat, polymere Epoxyde, Copolymere eines Alkylenoxyds und Alkylglycidylether.

Perorale osmotische Therapie-Systeme können jedoch neben dem Vorteil einer Kontrolle der Freisetzungsgeschwindigkeit den Nachteil eines Schädigungspotentials für die Magenschleimhaut aufweisen. Dabei können die Schädigungspotentiale der oft in hochkonzentrierter Form freigesetzten Wirkstoffe und Hilfsstoffe infolge der Fokussierung durch die kleine Austrittsöffnung erheblich vergrößert werden, so dass die Darmwand punktuell gravierend beschädigt wird.

Mit Vorteil ist dabei die Abgabeeinrichtung zur osmotisch gesteuerten Arzneistoff-freisetzung derart gestaltet, dass die Austrittsöffnung neben abstandshaltend gegenüber der Schleimhautoberfläche ausgebildeten Bereichen der Vorrichtung an einer relativ dazu zurückliegenden Stelle der äußeren Membran im Abstand zur Oberfläche der Schleimhaut angeordnet ist.

Dabei kann die Abgabeeinrichtung Vertiefungen, konkave Krümmungen, gekrümmte oder gewinkelte Achsen, Ringförmigkeit oder sonstige Gestaltungsmerkmale aufweisen, bei welchen direkter oder enger Kontakt der Austrittsöffnung mit der Oberfläche der den Magen auskleidenden Schleimhaut nicht möglich ist. Die Austrittsöffnung für die Arzneistofflösung ist dadurch zwangsweise im Abstand von Oberflächenregionen der Magenwand positioniert, indem zwischen der Austrittsöffnung und dem Teil der angrenzenden Magenwand ein Abstand eingehalten wird. Dieser Abstand fiihrt dazu, dass die Arzneistofflösung nicht in derselben hohen Konzentration, in welcher sie die Austrittsöffnung passiert, auf eine kleine, der Austrittsöffnung entsprechende Fläche der Magenschleimhaut trifft, sondern in verdünnter Form auf eine größere Fläche derselben. Arznei- oder Hilfsstoffe mit schleimhautschädigendem Potential erreichen nach Applikation eines erfindungsgemäßen gastroretentiven Systems mit einer derart gestalteten Abgabeeinrichtung die Magenschleimhaut nur nach vorheriger Verdünnung und können - wenn überhaupt - diese nur erheblich weniger schädigen als herkömmliche osmotische Therapie-Systeme.

Bei den Ausführungsformen des erfindungsgemäßen gastroretentiven Systems, bei dem die Abgabeeinrichtung zur osmotisch gesteuerten Arzneistoffabgabe ein Mehrkammer-System umfasst, liegen die Arzneistoffzubereitung und die osmotisch wirksame Substanz in getrennten Kammern innerhalb einer gemeinsamen Hülle vor, wobei die Kammer, welche die osmotisch wirksame Substanz enthält, keine Öffnung hat, die Arzneistoffzubereitung enthaltende Kammer jedoch eine nach außen führende, gegebenenfalls die gemeinsame Hülle durchdringende Öffnung für den Austritt des Arzneistoffs aufweist. Die Kammer, welche die osmotisch wirksame Substanz enthält, wird zumindest in einem Bereich von einer semipermeablen Membran begrenzt. Auch die Begrenzung der arzneistoffhaltigen Kammer muss zumindest in einem Bereich flexibel sein. Nach Applikation einer derartigen Abgabeeinrichtung führt die in die Kammer mit der osmotisch wirksamen Substanz eindringende Flüssigkeit zu einer Volumenvergrößerung dieser Kammer. Infolge ihrer Ausdehnung wird die Arzneistoffzubereitung durch die Öffnung aus seinem Kompartiment gedrückt und gelangt so in den Magen.

Bei einer anderen Ausführungsform, bei der die Arzneistoffabgabe erosionsgesteuert erfolgt, ist das erfindungsgemäße System wie eine Manteltablette aufgebaut, bei der der Quellkörper die Mantelschicht bildet, die den Tablettenkern umgibt. Der Tablettenkern besteht aus einer erodierbaren Masse, in der der Arzneistoff enthalten ist. Die den Tablettenkern umhüllende Mantelschicht weist eine Öffnung auf, durch welche Arzneistoff austreten kann. Die Freisetzungsrate des Arzneistoffs aus dem Tablettenkern, der die Abgabeeinrichtung dieses Systems darstellt, kann durch die Form und Größe des Tablettenkerns, beispielsweise durch Bildung einer sich mit zunehmendem Abstand zu der Öffnung vergrößernden Erosionsfront, sowie durch seine Löslichkeit/Erosionsgeschwindigkeit eingestellt werden (je nach Quellverhalten/Löslichkeit der für die Matrix verwendeten Polymere).

Der Begriff "Erosion" hat sich in der pharmazeutischen Technologie für alle Prozesse durchgesetzt, bei denen Feststoffmassen "abgetragen" werden. Dabei ist es nicht entscheidend, ob die Massenreduktion des festen Körpers dadurch zustande kommt, dass feste Bestandteile in Lösung gehen oder dass zunächst ein chemischer Abbau stattfindet, bei dem z. B. lange Polymerketten zu besser löslichen Oligomeren, Monomeren oder sonstigen Abbauprodukten gespalten werden.

Die erodierbare Masse besteht aus physiologisch unbedenklichen Polymeren oder wachsartigen Substanzen und gegebenenfalls weiteren pharmazeutischen Hilfsstoffen. Beispiele für solche Polymere sind Polysaccharide wie Gummen, Stärke- oder Cellulosederivate, Polyacrylate und -methacrylate, Polylaktide, Polyglykolide, Polyoxyethylene und Polyoxypropylene, Proteine, Polyvinylalkohol, Polyvinylacetat, Polyvinylchlorid oder Polyvinylpyrrolidon. Wachsartige Substanzen sind z. B. hydriertes Ricinusöl oder Cetylstearylalkohol. Weitere pharmazeutische Hilfsstoffe können aus den Gruppen der Stabilisatoren, Lösungsvermittler, Tenside, Füllstoffe, Weichmacher, Hydrophilisierungsmittel, Pigmente bzw. Farbstoffe, Substanzen zur Einstellung des pH-Wertes, Fließregulierungsmittel, Formtrennmittel, Schmiermittel usw. stammen. Je nach Kompatibilität und gewünschter Erosionsgeschwindigkeit muss der Anteil der einzelnen Komponenten eingestellt werden.

Die Erfindung umfasst auch Ausführungsformen für die erosionsgesteuerte Arzneistoffabgabe, bei denen die Abgabeeinrichtung in Form einer oder mehrerer Schichten vorliegen kann.

Das erfindungsgemäße gastroretentive System kann als zwei- oder mehrschichtige Tablette, oder als Manteltablette vorliegen. In einer bevorzugten Ausführungsform weist es eine Beschichtung auf, die sich im Magen zersetzt, oder es liegt in Form einer Kapsel vor, deren Hülle sich im Magen zersetzt. Diese Beschichtung bzw. Hülle soll das Schlucken des gastroretentiven Systems zumindest erleichtern.

Das erfindungsgemäße gastroretentive System hat im Besonderen den Vorteil, dass ein anderes Strukturelement die Rücksortierung des Systems im Magen sicherstellt als das, welches die kontinuierliche Wirkstoffabgabe bewirkt. Dadurch kann sichergestellt werden, dass der Rücksortiermechanismus des sich in der Verdauungsphase befindlichen Magens das erfindungsgemäße gastroretentive System als "noch nicht ausreichend zerkleinerten Nahrungsbestandteil" zurückhält und seinen Weitertransport in den Dünndarm vor Ende der vorgesehenen Dauer für die Abgabe des Arzneistoffs verhindert. Die Verweildauer des erfindungsgemäßen gastroretentiven Systems im Magen kann sich somit über die vorgesehene Dauer der Abgabe des Arzneistoffs erstrecken, die mindestens 4 Stunden beträgt, aber 24 Stunden nicht überschreiten und vorzugsweise zwischen 6 und 14 Stunden betragen soll.

### Beispiele

### Beispiel 1: Herstellung eines Quellkörpers

| Inhaltsstoff | Anteil |
|---|---|
| Natriumalginat | 59,1 Gew.-% |
| Mikrokristalline Cellulose | 39,4 Gew.-% |
| Magnesiumstearat | 1,0 Gew.-% |
| hochdisperses Siliciumdioxid | 0,5 Gew.-% |

Das Natriumalginat wurde mit der mikrokristallinen Cellulose mittels Polyvinylpyrrolidon-Lösung (Kollidon®30 in Ethanol) als Bindemittel granuliert. Nach dem Trocknen wurde das erhaltene Granulat klassiert (Korngröße 300 bis 800 nm). Zu dieser als innere Phase bezeichneten Mischung wurde die äußere Phase, bestehend aus Magnesiumstearat und hochdispersem Siliciumdioxid zugerüstet.

Anschließend erfolgte das Verpressen der Rezeptur mittels einer Tablettenpresse zu biplanen Tabletten mit einem Durchmesser von 13 mm und einer Masse von 600 mg.

### Beispiel 2: Herstellung eines Quellkörpers

| Inhaltsstoff | Anteil |
|---|---|
| Natriumalginat | 58,2 Gew.-% |
| Lactose | 38,8 Gew.-% |
| Calciumcarbonat | 1,5 Gew.-% |
| Magnesiumstearat | 1,0 Gew.-% |
| hochdisperses Siliciumdioxid | 0,5 Gew.-% |

Das Natriumalginat wurde mit der Lactose und dem Calciumcarbonat mittels Polyvinylpyrrolidon-Lösung (Kollidon®30 in Ethanol) als Bindemittel granuliert. Nach dem Trocknen wurde das erhaltene Granulat klassiert (Korngröße 300 bis 800 nm). Zu dieser als innere Phase bezeichneten Mischung wurde die äußere Phase, bestehend aus Magnesiumstearat und hochdispersem Siliciumdioxid zugerüstet.

Anschließend erfolgte das Verpressen der Rezeptur mittels einer Tablettenpresse zu biplanen Tabletten mit einem Durchmesser von 13 mm und einer Masse von 600 mg.

### Beispiel 3: Herstellung eines gastroretentiven Systems mit osmotisch gesteuerter Arzneistoff-Freisetzung

### Arzneistoffschicht:

| Inhaltsstoff | Anteil |
|---|---|
| Arzneistoff | 22,5 Gew.-% |
| Hydroxypropylmethylcellulose | 6,0 Gew.-% |
| Polyethylenoxid | 70,0 Gew.-% |
| Magnesiumstearat | 1,0 Gew.-% |
| hochdisperses Siliciumdioxid | 0,5 Gew.-% |

### Osmotisch wirksame Schicht:

| Inhaltsstoff | Anteil |
|---|---|
| Hydroxypropylmethylcellulose | 4,0 Gew.-% |
| Natriumchlorid | 30,0 Gew.-% |
| Polyethylenoxid | 65,0 Gew.-% |
| Magnesiumstearat | 1,0 Gew.-% |

### Überzug:

| Inhaltsstoff | Anteil |
|---|---|
| Celluloseacetat | 4,00 Gew.-% |
| Triethylcitrat | 0,14 Gew.-% |
| Polyethylenglykol | 2,00 Gew.% |
| Aceton/Isopropanol | 93,86 Gew.-% |
| (70:30, Angaben in Gew.-%) | |

### Quellkörper:

| Inhaltsstoff | Anteil |
|---|---|
| Natriumalginat | 53,5 Gew.-% |
| mikrokristalline Cellulose | 35,5 Gew.-% |
| Calciumcarbonat | 1,5 Gew.-% |
| quervernetzte Natriumcarboxymethylcellulose | 8,0 Gew.-% |
| Magnesiumstearat | 1,0 Gew.-% |
| hochdisperses Siliciumdioxid | 0,5 Gew.-% |

Die Bestandteile der inneren Phasen von Arzneistoffschicht, osmotisch wirksamer Schicht und Quellkörper wurden einzeln granuliert. Nach Zurüsten der äußeren Phase aus Magnesiumstearat bzw. Magnesiumstearat und hochdispersem Siliciumdioxid wurden die Arzneistoffschicht und die osmotisch wirksame Schicht zu einer bikonvexen Zweischichttablette verpresst, die anschließend mit dem Überzug versehen wurde. Der Überzug wurde im Bereich der Wirkstoffschicht mit einer Öffnung versehen, durch die der Arzneistoff aus dieser Abgabeeinrichtung abgegeben werden kann.

Das Granulat des Quellkörpers wurde nach Zurüsten der äußeren Phase zu einer einseitig konkav geformten Tablette verpresst und anschließend mit dieser Seite an die osmotisch wirksame Schicht der Abgabeeinrichtung geklebt.

### Beispiel 4: Herstellung eines gastroretentiven Systems mit erosionsgesteuerter Arzneistoff-Freisetzung

### Quellkörper (Mantelschicht):

| Inhaltsstoff | Anteil |
|---|---|
| Natriumalginat | 58,2 Gew.-% |
| Mikrokristalline Cellulose | 38,8 Gew.-% |
| Calciumcarbonat | 1,5 Gew.-% |
| Magnesiumstearat | 1,0 Gew.-% |
| hochdisperses Siliciumdioxid | 0,5 Gew.-% |

### Abgabeeinrichtung (Kern):

| Inhaltsstoff | Anteil |
|---|---|
| Arzneistoff | 58,0 Gew.-% |
| Hydroxypropylmethylcellulose | 7,5 Gew.-% |
| Lactose | 33,0 Gew.-% |
| Magnesiumstearat | 1,0 Gew.-% |
| hochdisperses Siliciumdioxid | 0,5 Gew.-% |

Die Bestandteile der inneren Phasen von Quellkörper und Abgabeeinrichtung wurden einzeln granuliert. Nach Zurüsten der äußeren Phase aus Magnesiumstearat und hochdispersem Siliciumdioxid zur inneren Phase der Abgabeeinrichtung wurde ein dreiecksförmiger spitzer Tablettenkern gepresst. Zur granulierten inneren Phase des Quellkörpers wurde ebenfalls die äußere Phase aus Magnesiumstearat und hochdispersem Siliciumdioxid zugerüstet. Anschießend wurde ein Teil des Granulats für die Mantelschicht der herzustellenden Manteltablette in die Matrize einer Tablettenpresse gefüllt, der zuvor gefertigte Tablettenkern darauf plaziert und mit dem restlichen Granulat für die Manteltablette aufgefüllt, so dass nach dem Verpressen eine bikonvexe Manteltablette erhalten wurde.

### Beispiel 5: Bestimmung des Quellverhaltens von Quellkörpern

Zur Bestimmung der Quelleigenschaften der verschiedenen Quellkörper wurden die relative Masseänderung von Quellkörpern, die gemäß Beispiel 1 (ohne Calcium) oder Beispiel 2 (mit Calcium) hergestellt wurden, bestimmt. Dazu wurden die Quellkörper in einem Dissolutionstester mit Blattrührer bei 50 Umdrehungen pro Minute in 37°C (±0,5°C) warmem Medium untersucht. Die relative Masseänderung ergibt sich aus der Formel (mₜ-m₀)/m₀, wobei m₀ die Masse des Quellkörpers zu Versuchsbeginn (Zeitpunkt t=0) und mₜ die Masse des Quellkörpers nach Inkubation in dem Medium für t Minuten ist.

Zunächst wurden die Quelleigenschaften von Quellkörpern bei konstantem pH-Wert des Mediums untersucht.

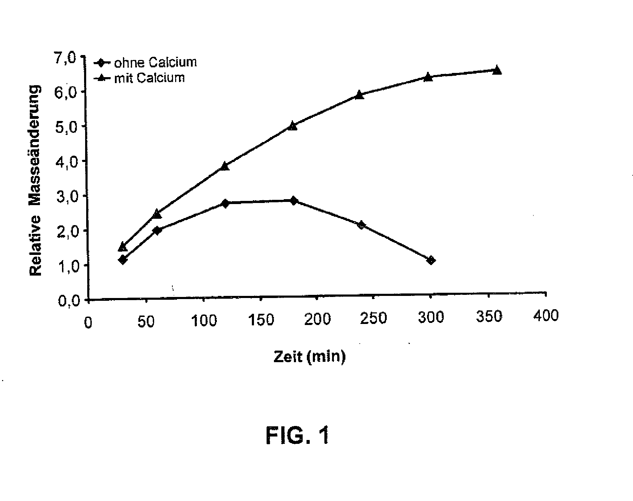

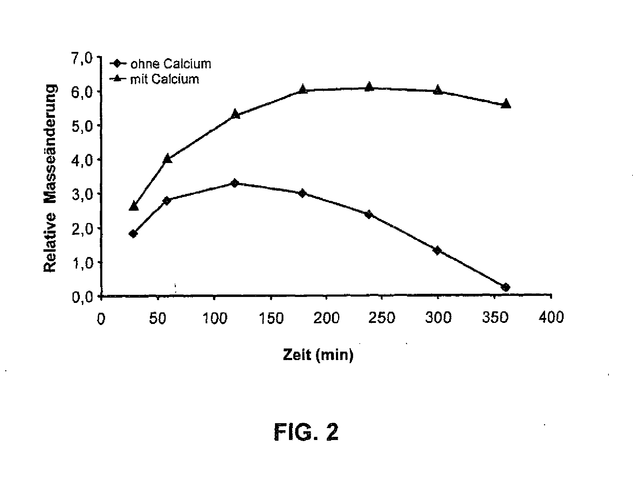

Die Ergebnisse dieser Untersuchungen sind in den Figuren 1 und 2 dargestellt. Das Medium wies entweder einen pH-Wert von 3 (Figur 1) oder einen pH-Wert von 4,5 (Figur 2) auf. Die Darstellungen dieser Versuchsergebnisse verdeutlichen die besseren Quelleigenschaften von Quellkörpern auf Basis von Natriumalginat, die Calciumionen enthalten, gegenüber calciumfreien Quellkörpern. Zudem zeigen die Quellkörper mit Calciumionen eine verbesserte Stabilität.

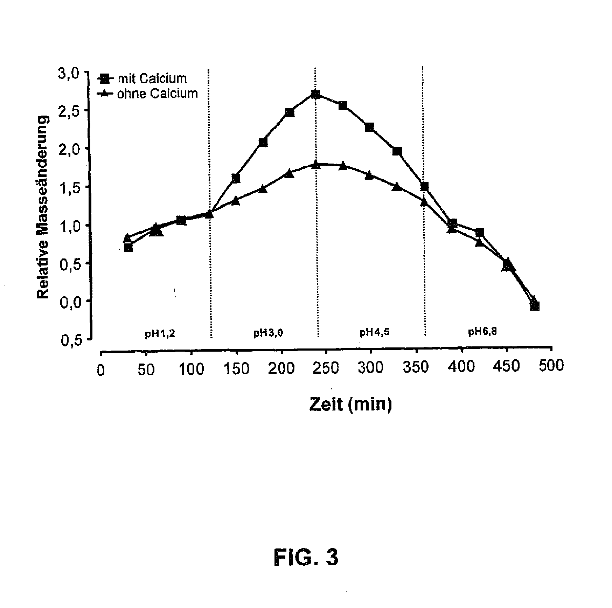

In einer weiteren Versuchsreihe wurden die Quelleigenschaften der Quellkörper bei wechselndem pH-Wert des Mediums untersucht. Dazu wurde der pH-Wert des Mediums schrittweise von 1,2 über 3,0 und 4,5 auf 6,8 erhöht. Die Ergebnisse dieser Versuche sind in Figur 3 dargestellt. Sie zeigen ebenfalls, dass der Zusatz von Calciumionen zum Natriumalginat die Quelleigenschaften des Quellkörpers verbessert. Zusätzlich wird deutlich, dass der Abbau gequollener Quellkörper bei höheren pH-Werten durch den Zusatz von Calciumionen nicht beeinträchtigt wird.

### Ausführungsformen

1. Gastroretentives System, umfassend
   a) mindestens eine Abgabeeinrichtung für mindestens einen pharmazeutischen Wirkstoff, und
   b) mindestens einen Quellkörper, der mit der Abgabeeinrichtung verbunden ist.
2. Gastroretentives System nach 1, dadurch gekennzeichnet, dass der Quellkörper auf einem Natriumalginat basiert.
3. Gastroretentives System nach 2, dadurch gekennzeichnet, dass dem Natriumalginat Calciumionen oder Calciumalginat zugesetzt sind/ist.
4. Gastroretentives System nach 3, dadurch gekennzeichnet, dass die Calciumionen in Form eines pharmazeutisch annehmbaren Calciumsalzes zugesetzt wurden, das vorzugsweise aus der Gruppe der Calciumsalze ausgewählt ist, die aus Calciumacetat, Calciumaspartat, Calciumcarbonat, Calciumchlorid, Calciumcitrat, Calciumcyclamat, Calciumfolinat, Calciumgluconat, Calciumglutamat, Calciumlactat, Calciumlactatgluconat, Calciumphosphat und Calciumsulfat besteht.
5. Gastroretentives System nach 4, dadurch gekennzeichnet, dass der Anteil an Calciumionen 0,1 bis 10 Gew.-%, vorzugsweise 0,3 bis 8 Gew.-%, besonders bevorzugt 0,5 bis 5 Gew.-% und ganz besonders bevorzugt 0,6 bis 2 Gew.-%, bezogen auf die Masse des Quellkörpers, beträgt.
6. Gastroretentives System nach einem von 2 bis 5, dadurch gekennzeichnet, dass dem Natriumalginat Zink- und/oder Aluminiumionen zugesetzt sind, vorzugsweise in Form eines oder mehrerer pharmazeutisch annehmbarer Zink- und/oder Aluminiumsalze.
7. Gastroretentives System nach 6, dadurch gekennzeichnet, dass die Zinkionen in Form eines pharmazeutisch annehmbaren Zinksalzes zugesetzt wurden, das vorzugsweise aus der Gruppe der Zinksalze ausgewählt ist, die aus Zinkacetat, Zinkaspartat, Zinkbishydrogenaspartat, Zinkchlorid und Zinkgluconat besteht, und dass die Aluminiumionen in Form eines pharmazeutisch annehmbaren Aluminiumsalzes zugesetzt wurden, das vorzugsweise aus der Gruppe der Aluminiumsalze ausgewählt ist, die aus Aluminiumhydroxid, Algedrat (Aluminiumoxid) und Aluminiumphosphat besteht.
8. Gastroretentives System nach 6 oder 7, dadurch gekennzeichnet, dass der Anteil an Zinksalz oder Aluminiumsalz zwischen 0,1 und 30 Gew.-%, vorzugsweise zwischen 1 und 25 Gew.-%, und besonders bevorzugt zwischen 5 und 15 Gew.-%, bezogen auf die Masse des Quellkörpers, beträgt.
9. Gastroretentives System nach einem von 2 bis 8, dadurch gekennzeichnet, dass der Quellkörper eine Mischung von Natriumalginat mit mindestens einem weiteren Polymer umfasst, das vorzugsweise aus der Gruppe der Polymere ausgewählt ist, die aus Croscarmellose-Natrium, Polycarbophil, Polyethylenoxid und Cellulosederivaten, vorzugsweise nicht wasserlöslichen Cellulosederivaten, insbesondere Ethylcellulose und Hydroxypropylmethylcellulose, besteht.
10. Gastroretentives System nach 9, dadurch gekennzeichnet, dass der Anteil an weiterem Polymer 1 bis 30 Gew.-%, vorzugsweise 3 bis 20 Gew.-%, besonders bevorzugt 5 bis 15 Gew.-%, bezogen auf die Masse des Quellkörpers, beträgt.
11. Gastroretentives System nach einem von 1 bis 10, dadurch gekennzeichnet, dass der Quellkörper mindestens einen pharmazeutisch annehmbaren Hilfsstoff aufweist, der vorzugsweise aus der Gruppe ausgewählt ist, die aus Füllstoffen, Bindemitteln, Fließregulierern, Schmiermitteln, Gleitmitteln, Trennmitteln und den pH-Wert beeinflussenden Stoffen besteht.
12. Gastroretentives System nach einem von 1 bis 11, dadurch gekennzeichnet, dass die Abgabeeinrichtung den Arzneistoff osmotisch gesteuert oder erosionsgesteuert abgeben kann.
13. Gastroretentives System nach 12, dadurch gekennzeichnet, dass die Abgabeeinrichtung ein osmotisches System mit einer Kammer ist, bei welchem ein arzneistoffhaltiger Kern, der gegebenenfalls zusätzlich zum Arzneistoff eine osmotisch wirksame Substanz enthält, von einer Wandung umgeben ist, die zumindest in einem Bereich eine semipermeable Membran aufweist, und in der sich mindestens eine Austrittsöffnung für den Arzneistoff befindet.
14. Gastroretentives System nach 12, dadurch gekennzeichnet, dass die Abgabeeinrichtung ein osmotisches System mit mindestens zwei Kammern ist, bei der Arzneistoff und osmotisch wirksame Substanz in getrennten Kammern innerhalb einer gemeinsamen Hülle vorliegen, wobei die Kammer, welche die osmotisch wirksame Substanz enthält, keine Öffnung hat, die Arzneistoffzubereitung enthaltende Kammer jedoch eine nach außen führende, gegebenenfalls die gemeinsame Hülle durchdringende Öffnung für den Austritt des Arzneistoffs aufweist, so dass nach Applikation des gastroretentiven Systems die in die Kammer mit der osmotisch wirksamen Substanz eindringende Flüssigkeit zu einer Volumenvergrößerung dieser Kammer führt, durch welche die Arzneistoffzubereitung durch die Öffnung aus seiner Kammer gedrückt wird.
15. Gastroretentives System nach 12, dadurch gekennzeichnet, dass die Abgabeeinrichtung eine arzneistoffhaltige, erodierbare Masse umfasst.
16. Gastroretentives System nach einem von 1 bis 15, dadurch gekennzeichnet, dass der Quellkörper und die Abgabeeinrichtung miteinander verpresst oder verklebt wurden, oder in einer gemeinsamen Hülle vorliegen.
17. Gastroretentives System nach einem von 1 bis 16, dadurch gekennzeichnet, dass es als zwei- oder mehrschichtige Tablette oder als Manteltablette vorliegt, die vorzugsweise mit einer sich im Magen auflösenden Beschichtung versehen sind, oder als Inhalt einer Kapsel, die das gastroretentive System im Magen freigibt.
18. Verfahren zur Herstellung eines gastroretetiven Systems nach einem von 1 bis 17, dadurch gekennzeichnet, dass der Quellkörper und die Abgabeeinrichtung miteinander verklebt oder verpresst, oder in einer gemeinsamen Hülle untergebracht werden.
19. Verwendung eines gastroretentiven Systems nach einem von 1 bis 17 zur kontrollierten Verabreichung eines Arzneistoffs.

## Patentansprüche

1. Gastroretentives System, das wie eine Manteltablette aufgebaut ist, umfassend
a) mindestens eine Abgabeeinrichtung für mindestens einen pharmazeutischen Wirkstoff, umfassend eine arzneistoffhaltige erodierbare Masse, und
b) mindestens einen Quellkörper, der auf einem Natriumalginat beruht und der mit der Abgabeeinrichtung verbunden ist,
**dadurch gekennzeichnet, dass** der Quellkörper eine Mantelschicht bildet, die den aus der erodierbaren Masse bestehenden Tablettenkern umgibt und eine Öffnung aufweist, durch welche Arzneistoff austreten kann.

2. Gastroretentives System nach Anspruch 1, **dadurch gekennzeichnet, dass** dem Natriumalginat Calciumionen oder Calciumalginat zugesetzt sind/ist.

3. Gastroretentives System nach Anspruch 2, **dadurch gekennzeichnet, dass** die Calciumionen in Form eines pharmazeutisch annehmbaren Calciumsalzes zugesetzt wurden, das vorzugsweise aus der Gruppe der Calciumsalze ausgewählt ist, die aus Calciumacetat, Calciumaspartat, Calciumcarbonat, Calciumchlorid, Calciumcitrat, Calciumcyclamat, Calciumfolinat, Calciumgluconat, Calciumglutamat, Calciumlactat, Calciumlactatgluconat, Calciumphosphat und Calciumsulfat besteht.

4. Gastroretentives System nach Anspruch 3, **dadurch gekennzeichnet, dass** der Anteil an Calciumionen 0,1 bis 10 Gew.-%, vorzugsweise 0,3 bis 8 Gew.-%, besonders bevorzugt 0,5 bis 5 Gew.-% und ganz besonders bevorzugt 0,6 bis 2 Gew.-%, bezogen auf die Masse des Quellkörpers, beträgt.

5. Gastroretentives System nach einem der Ansprüche 1 bis 4, **dadurch gekennzeichnet, dass** dem Natriumalginat Zink- und/oder Aluminiumionen zugesetzt sind, vorzugsweise in Form eines oder mehrerer pharmazeutisch annehmbarer Zink- und/oder Aluminiumsalze.

6. Gastroretentives System nach Anspruch 5, **dadurch gekennzeichnet, dass** die Zinkionen in Form eines pharmazeutisch annehmbaren Zinksalzes zugesetzt wurden, das vorzugsweise aus der Gruppe der Zinksalze ausgewählt ist, die aus Zinkacetat, Zinkaspartat, Zinkbishydrogenaspartat, Zinkchlorid und Zinkgluconat besteht, und das die Aluminiumionen in Form eines pharmazeutisch annehmbaren Aluminiumsalzes zugesetzt wurden, das vorzugsweise aus der Gruppe der Aluminiumsalze ausgewählt ist, die aus Aluminiumhydroxid, Algedrat (Aluminiumoxid) und Aluminiumphosphat besteht.

7. Gastroretentives System nach Anspruch 5 oder 6, **dadurch gekennzeichnet, dass** der Anteil an Zinksalz oder Aluminiumsalz zwischen 0,1 und 30 Gew.-%, vorzugsweise zwischen 1 und 25 Gew.-%, und besonders bevorzugt zwischen 5 und 15 Gew.-%, bezogen auf die Masse des Quellkörpers, beträgt.

8. Gastroretentives System nach einem der Ansprüche 1 bis 7, **dadurch gekennzeichnet, dass** der Quellkörper eine Mischung von Natriumalginat mit mindestens einem weiteren Polymer umfasst, das vorzugsweise aus der Gruppe der Polymere ausgewählt ist, die aus Croscarmellose-Natrium, Polycarbophil, Polyethylenoxid und Cellulosederivaten, vorzugsweise nicht wasserlöslichen Cellulosederivaten, insbesondere Ethylcellulose und Hydroxypropylmethylcellulose, besteht.

9. Gastroretentives System nach Anspruch 8, **dadurch gekennzeichnet, dass** der Anteil an weiterem Polymer 1 bis 30 Gew.-%, vorzugsweise 3 bis 20 Gew.-%, besonders bevorzugt 5 bis 15 Gew.-%, bezogen auf die Masse des Quellkörpers, beträgt.

10. Gastroretentives System nach einem der vorangehenden Ansprüche, **dadurch gekennzeichnet, dass** der Quellkörper mindestens einen pharmazeutisch annehmbaren Hilfsstoff aufweist, der vorzugsweise aus der Gruppe ausgewählt ist, die aus Füllstoffen, Bindemitteln, Fließregulierern, Schmiermitteln, Gleitmitteln, Trennmitteln und den pH-Wert beeinflussenden Stoffen besteht.

11. Gastroretentives System nach einem der vorangehenden Ansprüchen, **dadurch gekennzeichnet, dass** der Quellkörper und die Abgabeeinrichtung miteinander verpresst oder verklebt wurden, oder in einer gemeinsamen Hülle vorliegen.

12. Gastroretentives System nach einem der vorangehenden Ansprüche, **dadurch gekennzeichnet**, das es mit einer sich im Magen auflösenden Beschichtung versehen ist, oder als Inhalt einer Kapsel, die das gastroretentive System im Magen freigibt.

13. Verfahren zur Herstellung eines gastroretetiven Systems nach einem der vorangehenden Ansprüche, **dadurch gekennzeichnet, dass** der Quellkörper und die Abgabeeinrichtung miteinander verklebt oder verpresst, oder in einer gemeinsamen Hülle untergebracht werden.

14. Verwendung eines gastroretentiven Systems nach einem der Ansprüche 1 bis 12 zur kontrollierten Verabreichung eines Arzneistoffs.
